# EUROPÄISCHE PATENTSCHRIFT

(11) **EP 1 276 440 B1**
(45) Veröffentlichungstag und Bekanntmachung des Hinweises auf die Patenterteilung: **10.03.2004**
(21) Anmeldenummer: 01929613.6
(22) Anmeldetag: 02.05.2001
(51) Int. Cl.: A61F 2/36, A61B 17/86

(54) **SCHENKELHALSPROTHESE**
FEMORAL NECK PROSTHESIS
PROTHESE DU COL DU FEMUR

(30) Priorität: 03.05.2000 DE 10021527
(43) Veröffentlichungstag der Anmeldung: 22.01.2003
(73) Patentinhaber: Aesculap AG & Co. KG, 78532 Tuttlingen (DE)
(72) Erfinder: BLÖMER, Wilhelm, 78532 Tuttlingen (DE)
(74) Vertreter: Böhme, Ulrich, Dr. Dipl.-Phys.
(86) Internationale Anmeldenummer: PCT/EP2001/004911
(87) Internationale Veröffentlichungsnummer: WO 2001/082841

(56) Entgegenhaltungen:
- EP-A- 0 099 167
- EP-A- 0 158 014
- EP-A- 0 485 311
- WO-A-00/72785
- WO-A-01/13824
- WO-A-86/03962
- WO-A-92/12691
- WO-A-97/46179
- CH-A- 660 955
- DE-A- 3 607 824
- DE-U- 8 701 164
- DE-U- 20 007 950
- FR-A- 1 075 914
- FR-A- 1 105 392
- FR-A- 2 183 230
- FR-A- 2 595 565
- US-A- 4 005 495
- US-A- 5 571 203
- US-A- 5 741 262

## Beschreibung

Die Erfindung betrifft eine Schenkelhalsprothese mit einem Gelenkkopf und mit einem daran anschließenden schaftförmigen Verankerungsteil, dessen Längsachse im wesentlichen parallel zu einer Gelenkkopfachse verläuft, die durch eine Symmetrieachse einer kugelkalottenförmigen Gelenkfläche am Gelenkkopf gebildet wird.

Überwiegend werden für den künstlichen Hüftgelenkersatz im Femurbereich Schaftprothesen verwendet, die in den vorbereiten Markraum eingebracht werden (CH 660 955 A5). In diesen Fällen wird nach der Resektion des Femurkopfes der Markraum je nach Implantatform vorbereitet. Bei diesen Schaftprothesen wird der proximale Femurbereich relativ unphysiologisch beansprucht, was zu einer Veränderung des Knochens und somit zu einer Schaftlockerung führen kann. In diesem Fall muß eine Revisionsoperation durchgeführt werden, d. h die implantierte Prothese muß entfernt und durch eine neue Prothese ersetzt werden. Da aber bereits bei der Erstimplantation durch das Ausräumen des Markraumes ein nicht unerheblicher Teil des Knochenmaterials entfernt wurde, ist die Anpassung des Markraumes an die Form der neuen Prothese nicht ganz unproblematisch.

Neben den oben genannten Schaftprothesen gibt es auch sogenannte Schenkelhalsprothesen. Dabei handelt es sich um Prothesen, die mit einer geringfügigen Resektion natürlichen Knochenmaterials auskommen, der Stiel oder der Verankerungsschaft reichen in diesem Fall nicht in den Markraum.

Derartige Schenkelhalsprothesen sitzen lediglich im Schenkelhals des Femurs, die daraus resultierende Hüftgelenkskraft wird direkt über die Auflagefläche der Prothese in der Resektionsebene und über die Kortikalis des medialen Schenkelhalsstumpfes eingeleitet. Dadurch wird die physiologische Spannungsverteilung im proximalen Femurende weitgehend beibehalten.

Auch die Revision einer Schenkelhalsprothese ist wesentlich einfacher als dies bei einer Schaftprothese der Fall ist, da bei dem ersten Einsetzen einer derartigen Schenkelhalsprothese der größte Teil des Knochens erhalten bleiben kann.

Bekannte Schenkelhalsprothesen werden im lateralen Bereich des Femurknochens unterhalb des Trochanter Majors zusätzlich verankert. Hierzu wird der Femur in Richtung des Schenkelhalses vollständig durchbohrt, das Ende des schaftförmigen Verankerungsteils der Prothese wird dann über Platten und Schrauben an der lateralen Kortikalis befestigt (US-A-5 741 262; WO 98/06359; WO 86/03962; WO 92/12691).

Es ist auch bekannt, das schaftförmige Verankerungsteil an seinem freien Ende mit einem Gewinde zu versehen und dann direkt in die laterale Kortikalisbohrung einzuschrauben (WO 97/25939).

Nachteilig ist bei diesen bekannten Lösungen, daß die laterale Knochenstruktur erheblich geschwächt wird, und zwar in einem stark belasteten Bereich. Außerdem ist die Operationstechnik sehr kompliziert, da eine große Öffnung notwendig ist, um die laterale Befestigungslasche am Femur anbringen zu können.

Bei der Schenkelhalsprothese gemäß der WO-A-01/13824, welche einen Stand der Technik gemäß Art. 54(3)(4) darstellt, erfolgt die Stabilisierung des Verankerungsteils in einer Öffnung der Kortikalis, in diesem Falle durch eine zapfenförmige Verlängerung des Verankerungsteils, die durch die Kortikalis hindurchreicht. Auch in diesem Falle ist eine Schwächung der Kortikalis durch die Öffnung notwendig.

Es ist Aufgabe der Erfindung, eine gattungsgemäße Schenkelhalsprothese so auszugestalten, daß sie auch bei minimaler Schwächung des Knochens im Schenkelhalsbereich zuverlässig festgelegt werden kann.

Diese Aufgabe wird bei einer Schenkelhalsprothese der eingangs beschriebenen Art erfindungsgemäß dadurch gelöst, daß sie ein Abstützelement mit einer Fixierspitze und im Verankerungsteil in einem an dessen freies Ende anschließenden Bereich eine Aufnahmeausnehmung für die Fixierspitze des Abstützelementes umfaßt, in welche das etwa quer zur Längsrichtung des Verankerungsteiles verlaufende Abstützelement mit seiner Fixierspitze eintaucht, und daß in eine Sacklochbohrung des Verankerungsteiles ein Befestigungszapfen für den Gelenkkopf eingesetzt ist.

Bei dieser Konstruktion ist es möglich, eine Festlegung des schaftförmigen Verankerungsteils ausschließlich dadurch zu erreichen, daß in den Trochanter Major etwa quer zur Längsrichtung des Verankerungsteils verlaufend das Abstützelement eingesetzt wird, das dann mit seiner Fixierspitze in die Aufnahmeausnehmung des schaftförmigen Verankerungsteils eintritt und dadurch das Verankerungsteil zumindest in Längsrichtung fixiert.

Die Fixierspitze kann kugelkalottenförmig ausgebildet sein, so daß sie sich gegebenenfalls auch an unterschiedliche Winkel zwischen Abstützelement und Verankerungsteil anpaßt, außerdem erfolgt dadurch eine Zentrierung des Abstützelementes gegenüber dem Verankerungsteil beim Eintauchen der Fixierspitze in die Aufnahmeausnehmung.

Insbesondere kann auch vorgesehen sein, daß die Fixierspitze in einem sich an das freie Ende des Abstützelementes anschließenden Abschnitt konisch ausgebildet ist und sich zum freien Ende hin verjüngt.

Das Abstützelement kann beispielsweise die Form eines glatten oder strukturierten Schaftes aufweisen, besonders vorteilhaft ist es jedoch, wenn das Abstützelement eine Knochenschraube ist.

Günstig ist es dabei, wenn die Fixierspitze der Knochenschraube kein Außengewinde trägt, wenn also das Außengewinde der Knochenschraube in distaler Richtung vor der Fixierspitze aufhört.

Das Abstützelement kann insbesondere einen hohlen Schaft aufweisen.

Es ist dann günstig, wenn in der Wand des Abstützelementes Durchbrechungen angeordnet sind, so daß Knochenmaterial in das Innere des hohlen Schaftes hineinwachsen kann.

Die Aufnahmeausnehmung kann vom freien Ende des Verankerungsteils um 20 % bis 30 % der Länge des Verankerungsteils entfernt sein, so daß die Festlegung des Verankerungsteils durch das Abstützelement in der Nähe des freien Endes des Verankerungsteils erfolgt.

Bei einer ersten bevorzugten Ausführungsform ist vorgesehen, daß die Aufnahmeausnehmung als im Verankerungsteil angeordnete Vertiefung ausgebildet ist. Diese kann die Form einer Senkung aufweisen.

Der Querschnitt der Vertiefung kann bogenförmig ausgebildet sein, insbesondere kreisbogenförmig.

Bei einer anderen bevorzugten Ausführungsform ist die Aufnahmeausnehmung eine das Verankerungsteil quer durchsetzende Bohrung.

Es ist dabei vorteilhaft, wenn die Längsachse der Bohrung gegenüber der Längsachse des Verankerungsteils um 60° bis 80° geneigt ist, so daß entsprechend der Formgebung des Femurs ein Winkel zwischen diesen beiden Teilen eingehalten wird, der etwa dem Winkel zwischen dem Schenkelhals und dem Trochanter Major entspricht.

Günstig ist es, wenn die Bohrung konisch ausgebildet ist.

Insbesondere kann vorgesehen sein, daß bei einer Knochenschraube mit konischer Fixierspitze die Konizität des konischen Abschnittes und die Konizität der Bohrung gleich sind.

Bei einer weiteren bevorzugten Ausführungsform ist vorgesehen, daß die Aufnahmeausnehmung als um das Verankerungsteil umlaufende Umfangsnut ausgebildet ist.

Dabei ist es vorteilhaft, wenn das Abstützelement mit einem gabelförmig ausgebildeten vorderen Ende in die Umfangsnut eingreift.

Ein ganz wesentlicher Vorteil der beschriebenen Konstruktion liegt darin, daß auf eine Festlegung des Verankerungsteils an der lateralen Kortikalis verzichtet werden kann, so daß auch eine Durchbohrung des Femurs in diesem Bereich nicht notwendig ist. Das Verankerungsteil wird so kurz gewählt, daß es im Inneren des Femur angeordnet ist und die Kortikalis auf der dem Gelenkkopf gegenüberliegenden Seite des Femurs allenfalls erreicht, nicht aber durchbricht. Es ist dabei vorteilhaft, wenn die Länge des Verankerungsteils maximal 80 mm beträgt, insbesondere zwischen 20 mm und 70 mm.

Die Länge der Knochenschraube liegt vorzugsweise zwischen 15 mm und 40 mm.

Es ist vorteilhaft, wenn das Verankerungsteil eine Auflagefläche aufweist, die sich quer zu seiner Längsachse erstreckt. Dadurch wird ein Einsinken des Verankerungsteils in den Schenkelhals vermieden. Eine physiologische Krafteinleitung wird entsprechend bekannten Modellen erreicht.

Die Auflagefläche kann an ihrer Unterseite spitze Vorsprünge tragen, die zu einer zusätzlichen Festlegung der Auflagefläche an der Schnittfläche des Schenkelhalses beitragen.

Das Verankerungsteil kann massiv ausgebildet sein, günstig ist jedoch auch eine Ausbildung als Hohlschaft.

Weiterhin kann vorgesehen sein, daß das Verankerungsteil einen unrunden Querschnitt aufweist. Dadurch wird eine Verdrehung des Verankerungsteils um die Längsachse vermieden. Beispielsweise kann das Verankerungsteil im Querschnitt sternförmig ausgebildet sein.

Weiterhin ist es günstig, wenn das Verankerungsteil an seiner Oberfläche eine rauhe Mikrostruktur aufweist, dadurch wird das Einwachsen von Knochenmaterial gefördert.

Beispielsweise kann das Verankerungsteil an seiner Oberfläche mit einer mikroporösen Beschichtung versehen sein.

Bei einer bevorzugten Ausführungsform ist weiterhin vorgesehen, daß das Verankerungsteil mit einer bioaktiven Beschichtung versehen ist, insbesondere mit einer Beschichtung aus Kalziumphosphat, die elektrolytisch aufgebracht wird.

Bei einer bevorzugten Ausführungsform ist vorgesehen, daß der Befestigungszapfen in ein Sackloch des Gelenkkopfes eintaucht.

Insbesondere sind der Steckzapfen und das Sackloch konisch ausgebildet und weisen dieselbe Konizität auf. Es ist dadurch möglich, auf den Steckzapfen verschieden geformte Gelenkköpfe aufzusetzen, beispielsweise Gelenkköpfe mit unterschiedlichem Durchmesser, konzentrischem oder exzentrischem Sackloch oder unterschiedlicher Lage des Mittelpunkts der kugelkalottenförmigen Gelenkfläche.

Die nachfolgende Beschreibung bevorzugter Ausführungsformen der Erfindung dient im Zusammenhang mit der Zeichnung mit der Zeichnung der näheren Erläuterung.

Es zeigen:
- Figur 1:: eine Längsschnittansicht durch das Oberteil eines Femurs mit einer eingesetzten Schenkelhalsprothese und einer Aufnahmeausnehmung für eine Knochenschraube in Form einer Senkung;
- Figur 2:: eine Ansicht ähnlich Figur 1 mit einer Aufnahmeausnehmung in Form einer Querbohrung;
- Figur 3:: eine Teilansicht ähnlich Figur 1 mit einer Aufnahmeausnehmung in Form einer Umfangsnut und
- Figur 4:: eine Schnittansicht längs Linie 4-4 in Figur 3.

Die in der Zeichnung dargestellte Schenkelhalsprothese 1 umfaßt ein schaftförmiges Verankerungsteil 2, welches zylindrisch oder leicht konisch ausgebildet ist und welches sich an seinem proximalen Ende erweitert zu einer quer zu seiner Längsrichtung sich erstreckenden, flanschförmigen Auflagefläche 3, die im Querschnitt kreisförmig oder elliptisch ausgebildet sein kann und die an ihrer Unterseite in distaler Richtung weisende, spitze Vorsprünge 4 trägt. In eine zentrale Sacklochbohrung 5 in der Auflagefläche 3 ist ein Befestigungszapfen 6 eingesetzt, dessen proximales Ende eine sich zum freien Ende hin konisch verjüngende Verdickung 7 trägt.

Auf die konische Verdickung 7 des Befestigungszapfen 6 ist ein an seiner Außenseite eine kugelkalottenförmige Gelenkfläche 8 ausbildender Gelenkkopf 9 aufgesteckt, der zu diesem Zweck eine sich zu ihrem geschlossenen Ende hin konisch verjüngende Sacklochbohrung 10 aufweist, deren Konizität der der konischen Verdickung 7 des Befestigungszapfens 6 entspricht. Die Konizität ist dabei sehr gering und bewegt sich im Bereich einiger Grad, so daß beim Aufstecken des Gelenkkopfes 9 auf den Befestigungszapfen 6 Selbsthemmung eintritt.

Am gegenüberliegenden, distalen Ende endet das Verankerungsteil 2 in einer halbkugelförmigen Spitze 11, und etwa im Abstand von 20 % bis 30 % von dieser Spitze 11 ist in das Verankerungsteil 2 eine Senkung 12 eingearbeitet, die einen kreisbogenförmigen Querschnitt aufweist.

Die Schenkelhalsprothese 1 umfaßt weiterhin eine ein Abstützelement bildende Knochenschraube 13, die im dargestellten Ausführungsbeispiel als Hohlschraube ausgebildet ist mit einem Außengewinde 14, einem Eindrehkopf 15 und mit einer vom Außengewinde 14 freibleibenden, im dargestellten Ausführungsbeispiel halbkugelig endenden Fixierspitze 16. Im Bereich des Außengewindes 14 sind zwischen den Gewindegängen Durchbrechungen 17 in der Wand der Knochenschraube 13 vorgesehen, so daß vom Innenraum zum Außenraum der Knochenschraube 13 eine Anzahl von Verbindungen bestehen. Der Querschnitt der halbkugeligen Fixierspitze 16 entspricht dem Querschnitt der Senkung 12.

Zum Einsetzen der beschriebenen Schenkelhalsprothese, die aus einem körperverträglichen Metall besteht, beispielsweise aus Titan, wobei auch Gelenkköpfe aus Keramikmaterial oder Kunststoff Verwendung finden können, wird zunächst von einem Femur 18, in dessen Schenkelhals 19 die Schenkelhalsprothese 1 eingesetzt werden soll, der Gelenkkopf 20 durch einen quer zur Längsachse des Schenkelhalses 19 verlaufenden Schnitt 21 abgetrennt, und senkrecht zur Ebene dieses Schnittes 21 wird in den Schenkelhals 19 eine Bohrung 24 eingebracht, die bis in den Markraum 22 des Femurs 18 reicht, die dem Schenkelhals 19 gegenüberliegende Kortikalis 22 aber nicht erreicht.

In diese Bohrung 24 wird das schaftförmige Verankerungsteil 2 eingeschoben, bis die Auflagefläche 3 im Bereich des Schnittes 21 auf dem Schenkelhals 19 aufliegt, dabei dringen die spitzen Vorsprünge 4 in das Knochenmaterial ein und fixieren das Verankerungsteil 2 gegen eine Drehung um seine Längsachse.

Damit das Verankerungsteil 2 die Kortikalis 23 nicht erreicht, muß die Länge des Verankerungsteils 2 entsprechend bemessen sein, beispielsweise ist dieses Verankerungsteil nicht länger als 80 mm, vorzugsweise liegt die Länge im Bereich zwischen 20 mm und 70 mm.

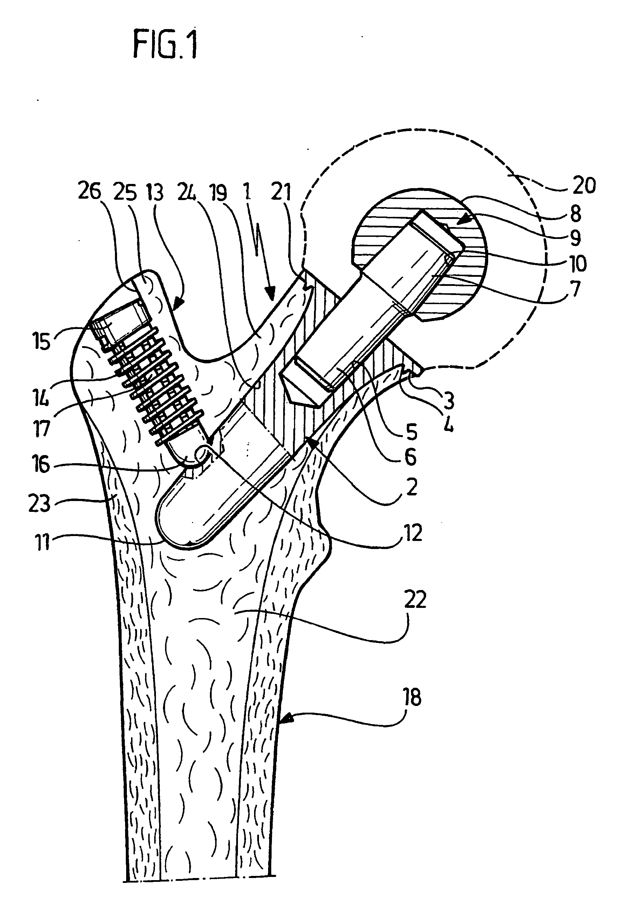

Zur Festlegung des Verankerungsteils 2 im Femur 18 wird nun durch eine längs des Trochanter Major 25 in den Femur 18 eingearbeitete Bohrung 26 die Knochenschraube 13 eingeschraubt, bis die halbkugelige Fixierspitze 16 in die Senkung 12 des Verankerungsteils 2 eintaucht, wie dies in Figur 1 dargestellt ist. Die Bohrung 26 wird zu diesem Zweck entsprechend positioniert, dabei verläuft die eingesetzte Knochenschraube 13 quer zur Längsrichtung des Verankerungsteils 2, die Längsachse der Knochenschraube 13 und die Längsachse des Verankerungsteils 2 schließen beispielsweise einen Winkel von 60 % bis 80 % miteinander ein. Durch das Eingreifen der Fixierspitze 16 in die Senkung 12 ergibt sich eine Fixierung des Verankerungsteils 2 in Längsrichtung, so daß auf diese Weise ohne Verletzung der lateralen Kortikalis eine sichere Festlegung des Verankerungsteils 2 im Femur 18 erreichbar ist.

Auf den im Verankerungsteil 2 gehaltenen Befestigungszapfen 6 wird dann ein Gelenkkopf 9 der gewünschten Abmessungen und Orientierung aufgesteckt, dieser Gelenkkopf 9 greift in eine in der Zeichnung nicht dargestellte Pfanne des Hüftgelenkes ein.

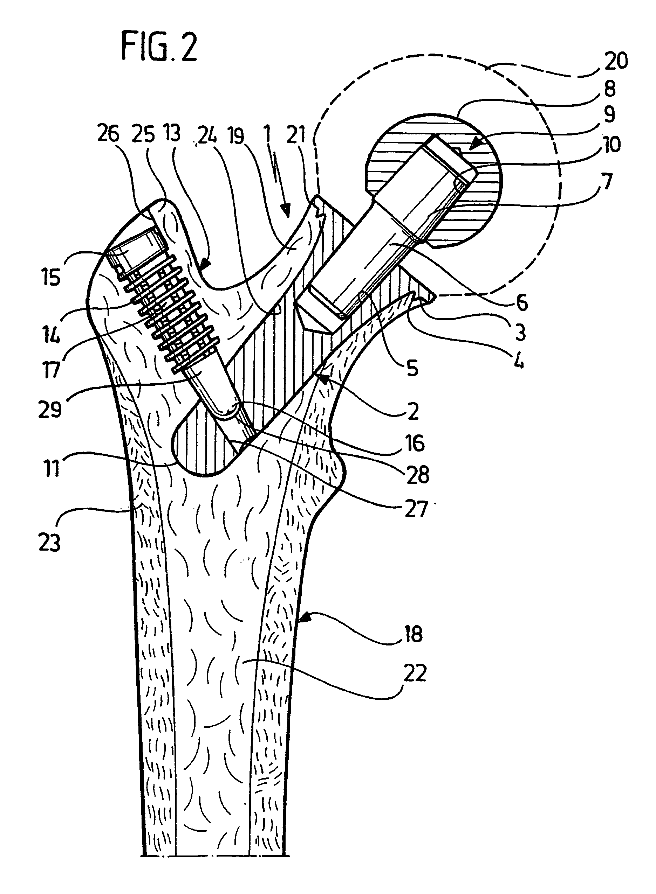

Bei dem Ausführungsbeispiel der Figur 2 ist ein sehr ähnlicher Aufbau gewählt, einander entsprechende Teile tragen daher dieselben Bezugszeichen.

Im Unterschied zum Ausführungsbeispiel der Figur 1 fehlt bei dem Verankerungsteil 2 dieser Schenkelhalsprothese 1 eine Senkung 12, statt dessen ist in der Verlängerung der Bohrung 24 eine das schaftförmige Verankerungsteil 2 durchsetzende Querbohrung 27 angeordnet, die leicht konisch ausgebildet ist und deren Längsachse gegenüber der Längsachse des Verankerungsteils 2 entsprechend der Ausrichtung der Bohrung 26 geneigt ist, beispielsweise um einen Winkel zwischen 60° und 80°.

Die Knochenschraube 13 ist in diesem Falle mit einer Fixierspitze 16 versehen, die neben einem balligen, kugelkalottenförmigen distalen Ende 28 in proximaler Richtung versetzt einen konischen Fixierbereich 29 aufweist, dessen Konizität der der Bohrung 26 entspricht, so daß beim Eintauchen dieser Fixierspitze 16 in die Querbohrung 27 eine Verriegelung des Verankerungsteiles 2 erfolgt, dieses ist durch diese Verriegelung nicht nur in axialer Richtung festgelegt, sondern auch gegen eine Drehung um die Längsachse gesichert.

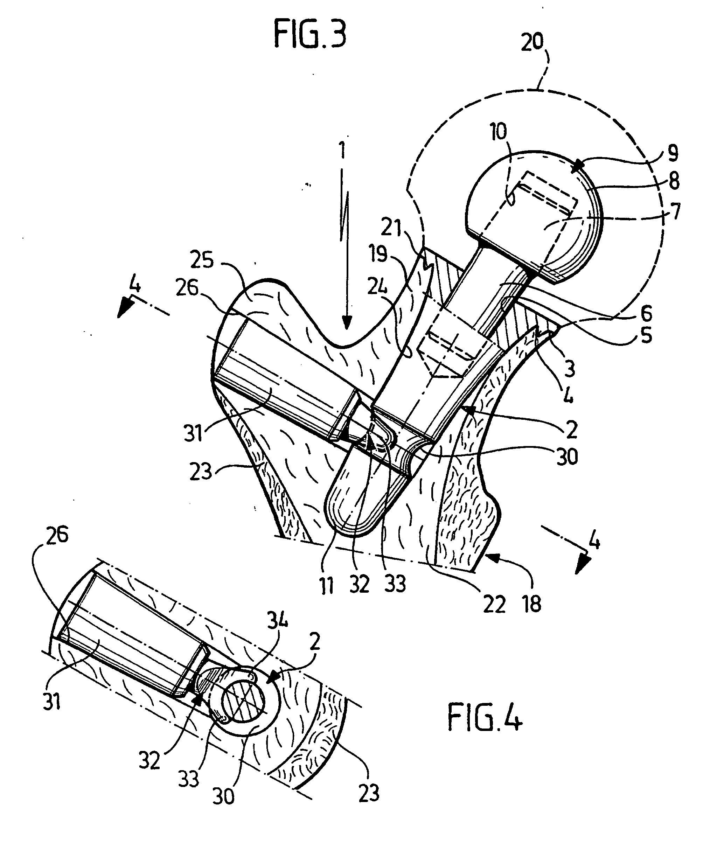

Das in den Figuren 3 und 4 dargestellte Ausführungsbeispiel entspricht ebenfalls weitgehend dem der voranstehenden Figuren 1 und 2, einander entsprechende Teile tragen daher dieselben Bezugszeichen.

Das Verankerungsteil 2 weist bei diesem Ausführungsbeispiel eine Aufnahmeausnehmung in Form einer Umfangsnut 30 auf, die in einer Ebene quer zur Längsrichtung des Verankerungsteils 2 umläuft und im Querschnitt bogenförmig ausgebildet ist.

Als Abstützelement ist in diesem Falle nicht eine Knochenschraube verwendet, sondern ein hohler Schaft 31, der ein gabelförmiges Ende 32 aufweist. Die beiden Zinken 33, 34 dieses gabelförmigen Endes 32 greifen in die Umfangsnut 30 ein und stützen sich am Boden dieser Umfangsnut 30 ab, so daß dadurch eine Festlegung des Verankerungsteils 2 zu erreichen ist.

Grundsätzlich wäre es aber auch möglich, das Abstützelement als Knochenschraube auszubilden, sofern das gabelförmige Ende gegenüber der Knochenschraube um die Längsachse derselben verdrehbar ausgeführt wird, so daß das gabelförmige Ende in die Umfangsnut eintauchen kann und dadurch eine Drehung der Knochenschraube nicht behindert wird.

## Patentansprüche

1. Schenkelhalsprothese mit einem Gelenkkopf (9) und mit einem daran anschließenden schaftförmigen Verankerungsteil (2), dessen Längsachse im wesentlichen parallel zu einer Gelenkkopfachse verläuft, die durch eine Symmetrieachse einer kugelkalottenförmigen Gelenkfläche (8) am Gelenkkopf (9) gebildet wird, **dadurch gekennzeichnet, daß** sie ein Abstützelement (13) mit einer Fixierspitze (16; 32) und im Verankerungsteil (2) in einem an dessen freies Ende (11) anschließenden Bereich eine Aufnahmeausnehmung (12; 27; 30) für die Fixierspitze (16; 32) des Abstützelementes (13) umfaßt, in welche das etwa quer zur Längsrichtung des Verankerungsteiles (2) verlaufende Abstützelement (13) mit seiner Fixierspitze (16; 32) eintaucht, und daß in eine Sacklochbohrung (5) des Verankerungsteiles (2) ein Befestigungszapfen (6) für den Gelenkkopf (9) eingesetzt ist.

2. Schenkelhalsprothese nach Anspruch 1, **dadurch gekennzeichnet daß** die Fixierspitze (16) kugelkalottenförmig ausgebildet ist.

3. Schenkelhalsprothese nach Anspruch 1 oder 2, **dadurch gekennzeichnet, daß** die Fixierspitze (16) in einem sich an das freie Ende (28) des Abstützelementes (13) anschließenden Abschnitt (29) konisch ausgebildet ist und sich zum freien Ende (28) hin verjüngt.

4. Schenkelhalsprothese nach einem der voranstehenden Ansprüche, **dadurch gekennzeichnet, daß** das Abstützelement eine Knochenschraube (13) ist.

5. Schenkelhalsprothese nach Anspruch 4, **dadurch gekennzeichnet, daß** die Fixierspitze (16) der Knochenschraube (13) kein Außengewinde (14) trägt.

6. Schenkelhalsprothese nach einem der voranstehenden Ansprüche, **dadurch gekennzeichnet, daß** das Abstützelement (13) einen hohlen Schaft aufweist.

7. Schenkelhalsprothese nach Anspruch 6, **dadurch gekennzeichnet, daß** in der Wand des Abstützelementes (13) Durchbrechungen (17) angeordnet sind.

8. Schenkelhalsprothese nach einem der voranstehenden Ansprüche, **dadurch gekennzeichnet, daß** die Aufnahmeausnehmung (12; 27) vom freien Ende (11; 28) des Verankerungsteils (2) um 20 % bis 30 % der Länge des Verankerungsteils (2) entfernt ist.

9. Schenkelhalsprothese nach einem der voranstehenden Ansprüche, **dadurch gekennzeichnet, daß** die Aufnahmeausnehmung als im Verankerungsteil (2) angeordnete Vertiefung (12) ausgebildet ist.

10. Schenkelhalsprothese nach Anspruch 9, **dadurch gekennzeichnet, daß** der Querschnitt der Vertiefung (12) bogenförmig ausgebildet ist.

11. Schenkelhalsprothese nach einem der Ansprüche 1 bis 8, **dadurch gekennzeichnet, daß** die Aufnahmeausnehmung eine das Verankerungsteil (2) quer durchsetzende Bohrung (27) ist.

12. Schenkelhalsprothese nach Anspruch 11, **dadurch gekennzeichnet, daß** die Längsachse der Bohrung (27) gegenüber der Längsachse des Verankerungsteils (2) um 60° bis 80° geneigt ist.

13. Schenkelhalsprothese nach Anspruch 11 oder 12, **dadurch gekennzeichnet, daß** die Bohrung (27) konisch ausgebildet ist.

14. Schenkelhalsprothese nach Anspruch 13 und nach Anspruch 3, **dadurch gekennzeichnet, daß** die Fixierspitze (13) in ihrem konischen Abschnitt (29) und die Bohrung (27) die gleiche Konizität aufweisen.

15. Schenkelhalsprothese nach einem der Ansprüche 1 bis 8, **dadurch gekennzeichnet, daß** die Aufnahmeausnehmung als im Verankerungsteil (2) umlaufende Umfangsnut (30) ausgebildet ist.

16. Schenkelhalsprothese nach Anspruch 15, **dadurch gekennzeichnet, daß** das Abstützelement (13) mit einem gabelförmig ausgebildeten vorderen Ende (32) in die Umfangsnut (30) eingreift.

17. Schenkelhalsprothese nach einem der voranstehenden Ansprüche, **dadurch gekennzeichnet, daß** die Länge des Verankerungsteils (2) maximal 80 mm beträgt.

18. Schenkelhalsprothese nach Anspruch 17, **dadurch gekennzeichnet, daß** die Länge des Verankerungsteils (2) zwischen 20 mm und 70 mm liegt.

19. Schenkelhalsprothese nach einem der voranstehenden Ansprüche, **dadurch gekennzeichnet, daß** die Länge des Abstützelementes (13) zwischen 15 mm und 40 mm liegt.

20. Schenkelhalsprothese nach einem der voranstehenden Ansprüche, **dadurch gekennzeichnet, daß** das Verankerungsteil (2) eine Auflagefläche (3) aufweist, die sich quer zu seiner Längsachse erstreckt.

21. Schenkelhalsprothese nach Anspruch 20, **dadurch gekennzeichnet, daß** die Auflagefläche (3) an ihrer Unterseite spitze Vorsprünge (4) trägt.

22. Schenkelhalsprothese nach einem der voranstehenden Ansprüche, **dadurch gekennzeichnet daß** der Befestigungszapfen (6) in ein Sackloch (10) des Gelenkkopfes (9) eintaucht.

23. Schenkelhalsprothese nach Anspruch 22, **dadurch gekennzeichnet, daß** der Steckzapfen (6) und das Sackloch (10) konisch ausgebildet sind und dieselbe Konizität aufweisen.

24. Schenkelhalsprothese nach einem der voranstehenden Ansprüche, **dadurch gekennzeichnet, daß** das Verankerungsteil (2) als Hohlschaft ausgebildet ist.

25. Schenkelhalsprothese nach einem der voranstehenden Ansprüche, **dadurch gekennzeichnet, daß** das Verankerungsteil (2) einen unrunden Querschnitt aufweist.

26. Schenkelhalsprothese nach Anspruch 25, **dadurch gekennzeichnet, daß** das Verankerungsteil (2) im Querschnitt sternförmig ausgebildet ist.

27. Schenkelhalsprothese nach einem der voranstehenden Ansprüche, **dadurch gekennzeichnet, daß** das Verankerungsteil (2) an seiner Oberfläche eine rauhe Mikrostruktur aufweist.

28. Schenkelhalsprothese nach Anspruch 27, **dadurch gekennzeichnet, daß** das Verankerungsteil (2) an seiner Oberfläche mit einer mikroporösen Beschichtung versehen ist.

29. Schenkelhalsprothese nach einem der voranstehenden Ansprüche, **dadurch gekennzeichnet, daß** das Verankerungsteil (2) mit einer bioaktiven Beschichtung versehen ist.

30. Schenkelhalsprothese nach Anspruch 29, **dadurch gekennzeichnet, daß** das Verankerungsteil mit einer Beschichtung aus Kalziumphosphat versehen ist.

## Claims

1. A femoral neck prosthesis comprising a joint head (9) and a shaft-type anchoring part (2) connected thereto, the longitudinal axis of the anchoring part (2) extending substantially parallel to a joint-head axis formed by an axis of symmetry of a spherical cap-shaped joint surface (8) on the joint head (9), **characterised in that** it includes a supporting component (13) with a fixing tip (16; 32) and, in the anchoring part (2) in a region adjacent to its free end (11), a holding recess (12; 27; 30) for the fixing tip (16; 32) of the supporting component (13), which extends substantially transversely to the longitudinal direction of the anchoring part (2) and projects with its fixing tip (16; 32) into the holding recess (12; 27; 30), and **in that** a fixing pin (6) for the joint head (9) is inserted into a blind hole (5) in the anchoring part (2).

2. A femoral neck prosthesis according to claim 1, **characterised in that** the fixing tip (16) is formed in the shape of a spherical cap.

3. A femoral neck prosthesis according to claim 1 or 2, **characterised in that** the fixing tip (16) is conically formed in a portion (29) adjacent to the free end (28) of the supporting component (13) and tapers towards the free end (28).

4. A femoral neck prosthesis according to any one of the preceding claims, **characterised in that** the supporting component is a bone screw (13).

5. A femoral neck prosthesis according to claim 4, **characterised in that** the fixing tip (16) of the bone screw (13) does not have an external thread (14).

6. A femoral neck prosthesis according to any one of the preceding claims, **characterised in that** the supporting component (13) has a hollow shaft.

7. A femoral neck prosthesis according to claim 6, **characterised in that** openings (17) are arranged in the wall of the supporting component (13).

8. A femoral neck prosthesis according to any one of the preceding claims, **characterised in that** the holding recess (12; 27) is spaced from the free end (11; 28) of the anchoring part (2) by 20% to 30% of the length of the anchoring part (2).

9. A femoral neck prosthesis according to any one of the preceding claims, **characterised in that** the holding recess is formed as a depression (12) provided in the anchoring part (2).

10. A femoral neck prosthesis according to claim 9, **characterised in that** the depression (12) is curved in cross-section.

11. A femoral neck prosthesis according to any one of claims 1 to 8, **characterised in that** the holding recess is a bore (27) extending transversely through the anchoring part (2).

12. A femoral neck prosthesis according to claim 11, **characterised in that** the longitudinal axis of the bore (27) is inclined at an angle of 60° to 80° to the longitudinal axis of the anchoring part (2).

13. A femoral neck prosthesis according to claim 11 or 12, **characterised in that** the bore (27) is conically formed.

14. A femoral neck prosthesis according to claim 13 and claim 3, **characterised in that** the bore (27) and the conical portion (29) of the fixing tip (13) have the same conicity.

15. A femoral neck prosthesis according to any one of claims 1 to 8, **characterised in that** the holding recess is formed as a circumferential groove (30) extending round the anchoring part (2).

16. A femoral neck prosthesis according to claim 15, **characterised in that** the supporting component (13) has a fork-shaped front end (32) engaging in the circumferential groove (30).

17. A femoral neck prosthesis according to any one of the preceding claims, **characterised in that** the length of the anchoring part (2) is at most 80 mm.

18. A femoral neck prosthesis according to claim 17, **characterised in that** the length of the anchoring part (2) lies between 20 mm and 70 mm.

19. A femoral neck prosthesis according to any one of the preceding claims, **characterised in that** the length of the supporting component (13) lies between 15 mm and 40 mm.

20. A femoral neck prosthesis according to any one of the preceding claims, **characterised in that** the anchoring part (2) has a supporting surface (3) extending transversely to its longitudinal axis.

21. A femoral neck prosthesis according to claim 20, **characterised in that** the supporting surface (3) carries pointed projections (4) on its underside.

22. A femoral neck prosthesis according to any one of the preceding claims, **characterised in that** the fixing pin (6) projects into a blind hole (10) in the joint head (9).

23. A femoral neck prosthesis according to claim 22, **characterised in that** the insertion pin (6) and the blind hole (10) are conically formed and have the same conicity.

24. A femoral neck prosthesis according to any one of the preceding claims, **characterised in that** the anchoring part (2) is formed as a hollow shaft.

25. A femoral neck prosthesis according to any one of the preceding claims, **characterised in that** the anchoring part (2) has a non-circular cross-section.

26. A femoral neck prosthesis according to claim 25, **characterised in that** the anchoring part (2) is star-shaped in cross-section.

27. A femoral neck prosthesis according to any one of the preceding claims, **characterised in that** the anchoring part (2) has a rough microstructure on its surface.

28. A femoral neck prosthesis according to claim 27, **characterised in that** the surface of the anchoring part (2) is provided with a microporous coating.

29. A femoral neck prosthesis according to any one of the preceding claims, **characterised in that** the anchoring part (2) is provided with a bioactive coating.

30. A femoral neck prosthesis according to claim 29, **characterised in that** the anchoring part is provided with a calcium phosphate coating.

## Revendications

1. Prothèse du col du fémur, comportant une tête d'articulation (9) avec une partie d'ancrage (2) en forme de fût raccordée à celle-ci, dont l'axe longitudinal s'étend sensiblement parallèlement à un axe de tête d'articulation qui est formé par un axe de symétrie d'une surface d'articulation (8) en forme de calotte sphérique sur la tête d'articulation (9), **caractérisée en ce qu'**elle comprend un élément d'appui (13) avec une pointe de fixation (16 ; 32) et un évidement de réception (12 ; 27 ; 30) situé dans la partie d'ancrage (2) dans une zone se raccordant à son extrémité libre (11), pour la pointe de fixation (16 ; 32) de l'élément d'appui (13), dans lequel l'élément d'appui (13) s'étendant approximativement transversalement à la direction longitudinale de la partie d'ancrage (2) plonge avec sa pointe de fixation (16 ; 32), et **en ce qu'**un pivot de fixation (6) pour la tête d'articulation (9) est inséré dans un trou borgne (5) de la partie d'ancrage'(2).

2. Prothèse du col du fémur selon la revendication 1, **caractérisée en ce que** la pointe de fixation (16) est réalisée en forme de calotte sphérique.

3. Prothèse du col du fémur selon l'une ou l'autre des revendications 1 et 2, **caractérisée en ce que** la pointe de fixation (16) est réalisée conique dans un tronçon (29) se raccordant à l'extrémité libre (28) de l'élément d'appui (13) et se rétrécit vers l'extrémité libre (28).

4. Prothèse du col du fémur selon l'une des revendications précédentes, **caractérisée en ce que** l'élément d'appui est une vis à os (13).

5. Prothèse du col du fémur selon la revendication 4, **caractérisée en ce que** la pointe de fixation (16) de la vis à os (13) ne porte aucun pas de vis extérieur (14).

6. Prothèse du col du fémur selon l'une des revendications précédentes, **caractérisée en ce que** l'élément d'appui (13) présente un fût creux.

7. Prothèse du col du fémur selon la revendication 6, **caractérisée en ce que** des percées (17) sont ménagées dans la paroi de l'élément d'appui (13).

8. Prothèse du col du fémur selon l'une des revendications précédentes, **caractérisée en ce que** l'évidement de réception (12 ; 27) est éloigné de l'extrémité libre (11 ; 28) de la partie d'ancrage (2) de 20 % à 30 % de la longueur de la partie d'ancrage (2).

9. Prothèse du col du fémur selon l'une des revendications précédentes, **caractérisée en ce que** l'évidement de réception est réalisé sous forme de renfoncement (12) agencé dans la partie d'ancrage (2).

10. Prothèse du col du fémur selon la revendication 9, **caractérisée en ce que** la section transversale du renfoncement (12) est réalisée en forme d'arc.

11. Prothèse du col du fémur selon l'une des revendications 1 à 8, **caractérisée en ce que** l'évidement est un perçage (27) traversant transversalement la partie d'ancrage (2).

12. Prothèse du col du fémur selon la revendication 11, **caractérisée en ce que** l'axe longitudinal du perçage (27) est incliné de 60° à 80° par rapport à l'axe longitudinal de la partie d'ancrage (2).

13. Prothèse du col du fémur selon l'une ou l'autre des revendications 11 et 12, **caractérisé en ce que** le perçage (27) est réalisé conique.

14. Prothèse du col du fémur selon la revendication 13 et selon la revendication 3, **caractérisée en ce que** la pointe de fixation (16), dans son tronçon conique (29), et le perçage (27) présentent la même conicité.

15. Prothèse du col du fémur selon l'une des revendications 1 à 8, **caractérisé en ce que** l'évidement de réception est réalisé sous forme de gorge périphérique (30) dans la partie d'ancrage (2).

16. Prothèse du col du fémur selon la revendication 15, **caractérisée en ce que** l'élément d'appui (13) s'engage avec une extrémité antérieure (32) réalisée en forme de fourche dans la gorge périphérique (30).

17. Prothèse du col du fémur selon l'une des revendications précédentes, **caractérisée en ce que** la longueur de la partie d'ancrage (2) est au maximum de 80 mm.

18. Prothèse du col du fémur selon la revendication 17, **caractérisée en ce que** la longueur de la partie d'ancrage (2) est entre 20 mm et 70 mm.

19. Prothèse du col du fémur selon l'une des revendications précédentes, **caractérisée en ce que** la longueur de l'élément d'appui (13) est entre 15 mm et 40 mm.

20. Prothèse du col du fémur selon l'une des revendications précédentes, **caractérisée en ce que** la partie d'ancrage (2) présente une surface d'appui (3) qui s'étend transversalement à son axe longitudinal.

21. Prothèse du col du fémur selon la revendication 20, **caractérisée en ce que** la surface d'appui (3) porte des saillies (4) pointues sur sa face inférieure.

22. Prothèse du col du fémur selon l'une des revendications précédentes, **caractérisée en ce que** le pivot de fixation (6) plonge dans un trou borgne (10) de la tête d'articulation (9).

23. Prothèse du col du fémur selon la revendication 22, **caractérisée en ce que** le pivot d'enfichage (6) et le trou borgne (10) sont réalisés coniques et présentent la même conicité.

24. Prothèse du col du fémur selon l'une des revendications précédentes, **caractérisée en ce que** la partie d'ancrage (2) est réalisée sous forme de fût creux.

25. Prothèse du col du fémur selon l'une des revendications précédentes, **caractérisée en ce que** la partie d'ancrage (2) présente une section transversale non ronde.

26. Prothèse du col du fémur selon la revendication 25, **caractérisée en ce que** la partie d'ancrage (2) est réalisé avec une section transversale en forme d'étoile.

27. Prothèse du col du fémur selon l'une des revendications précédentes, **caractérisée en ce que** la partie d'ancrage (2) présente à sa surface une microstructure rugueuse.

28. Prothèse du col du fémur selon la revendication 27, **caractérisée en ce que** la partie d'ancrage (2) est pourvue d'un revêtement microporeux sur sa surface.

29. Prothèse du col du fémur selon l'une des revendications précédentes, **caractérisée en ce que** la partie d'ancrage (2) est pourvue d'un revêtement bioactif.

30. Prothèse du col du fémur selon la revendication 29, **caractérisée en ce que** la partie d'ancrage est pourvue d'un revêtement en phosphate de calcium.
